# EUROPÄISCHE PATENTSCHRIFT

(11) **EP 0 612 322 B1**
(45) Veröffentlichungstag und Bekanntmachung des Hinweises auf die Patenterteilung: **03.03.1999**
(21) Anmeldenummer: 92923141.3
(22) Anmeldetag: 03.11.1992
(51) Int. Cl.: C07D 475/04

(54) **VERFAHREN ZUR TRENNUNG VON 5-METHYL-TETRAHYDROFOLSÄURE**
METHOD OF SEPARATING THE ENANTIOMERS OF 5-METHYL-TETRAHYDROFOLIC ACID
PROCEDE DE SEPARATION DE L'ACIDE 5-METHYL-TETRAHYDROFOLLIQUE

(30) Priorität: 11.11.1991 DE 4136921
(43) Veröffentlichungstag der Anmeldung: 31.08.1994
(73) Patentinhaber: KNOLL AKTIENGESELLSCHAFT, D-67061 Ludwigshafen (DE)
(72) Erfinder: SCHEIB, Klaus, D-6701 Schauernheim (DE); KLEIN, Peter, D-6701 Birkenheide (DE); CARTER, Robert, CH-4054 Basel (CH)
(74) Vertreter: Karau, Wolfgang, Dr.
(86) Internationale Anmeldenummer: EP9202515
(87) Internationale Veröffentlichungsnummer: WO9310118

(56) Entgegenhaltungen:
- EP-A- 0 266 042
- EP-A- 0 293 029
- EP-A- 0 348 641
- EP-A- 0 367 902
- EP-A- 0 432 441
- EP-A- 0 455 013
- EP-A- 0 495 204
- WO-A-91/13890

## Beschreibung

Die vorliegende Erfindung betrifft ein Verfahren zur Trennung von (6 R,S)-5-Methyl-tetrahydrofolsäure der Formel

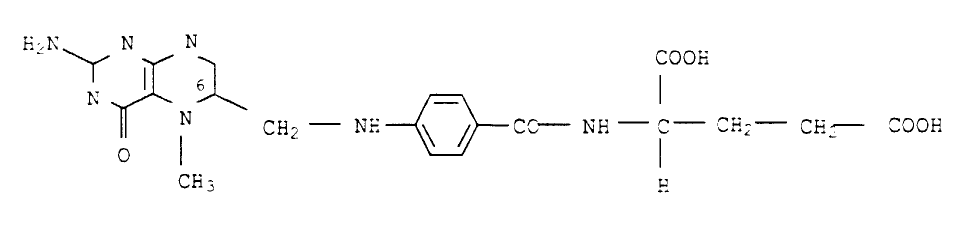

Diese Säure wird auch als N-(5-Methyl)-(6R,S)-5,6,7,8-tetrahydropteroyl)-L-glutaminsäure bezeichnet. Sie wird aus Folsäure durch Methylierung und Hydrierung hergestellt und galt lange Zeit als auf chemischem Weg nicht in seine Diastereomeren trennbar (Clinical Science and Molecular Medicine 45, 625-631 (1973)). Erst 1991 ist ein Verfahren zur Trennung von Ammoniumsalzen der N-(5-Methyl)-(6R,S)-5,6,7,8-tetrahydropteroyl)-L-glutaminsäure durch fraktionierte Kristallisation beschrieben worden (EP-A-455 013). Das Verfahren ist jedoch sehr aufwendig, da aus dem Ammoniumsalz der N-(5-Methyl)-(6R,S)-5,6,7,8-tetrahydropteroyl)-L-glutaminsäure zunächst das 6R-Isomere isoliert werden muß. Aus den erhaltenen Mutterlaugen wird anschließend das 6S-Isomere gewonnen.

Weiter ist ein Verfahren zur Racemattrennung von Folinsäure bekannt (EP-A-293 029), bei dem das Calcium-Salz des (S)-Isomeren in drei unterschiedlichen Kristallisationsschritten aus dem Isomerengemisch isoliert wird.

5-Methyl-tetrahydrofolsäure hat Bedeutung als Arzneimittelwirkstoff in 2 Hauptindikationsgebieten:
a) in der Onkologie als Co-Therapie bei der Methotrexat- und 5-Fluorouracil-Behandlung und
b) bei der Behandlung einer Folsäuremangel-Anämie bei Schwangerschaft, Antibiotika-Therapien u.a.

Ca-5-Methyl-tetrahydrofolat ist das einzige Folsäurederivat auf dem Markt, das direkt die Blut-Hirn-Schranke ohne weitere Metabolisierung durchbrechen kann.

In natürlichen Vorkommen findet man 5-Methyl-tetrahydrofolsäure nur in der S-Form, die R-Form ist biochemisch unwirksam und wird durch die Niere ausgeschieden.

Es wurde nun ein Verfahren gefunden, mit dessen Hife man die Säure in ihrer Diastereomeren trennen kann. Gegenstand der Erfindung ist ein Verfahren zur Herstellung von (6S)-5-Methyl-tetrahydrofolsäure durch Racemattrennung der (6R,S)-S-Methyl-tetrahydrofolsäure mit Hilfe einer Base, dadurch gekennzeichnet, daß man als Base N-Ethyl-2-aminomethylpyrrolidin oder dessen optische Isomere verwendet.

Zur Durchführung des Verfahrens wird die Säure oder eine wasserlösliches Salt derselben in Wasser aufgenommen und mit einer Lösung von N-Ethyl-2-aminomethyl-pyrrolidin bzw. einem optischen Isomeren dieser Verbindung in Wasser versetzt. Dabei fällt das Salz der Säure mit dem Pyrrolidin-Derivat aus. Erwärmt man das Reaktionsgemisch auf 40 - 90, vorzugsweise 60 - 80°C, geht das Salz der 6S-Säure in Lösung und kann so von dem Salz der 6R-Säure abgetrennt werden. Beim Abkühlen der Lösung fällt das Salz der 6S-Säure wieder aus.

Aus letzterem erhält man durch Aufschlämmen in Wasser und Zugabe von Natronlauge das Natriumsalz, das mit beispielsweise einem Erdalkalihydroxid bzw. -chlorid in eine Erdalkalisalz wie das Calcium-Salz, übergeführt werden kann.

Das N-Ethyl-2-aminomethylpyrrolidin kann wie bereits erwähnt sowohl als Racemat als auch in Form seiner optischen Isomere für die Racemattrennung verwendet werden. Am besten geeignet ist das (-)-drehende Amin.

### Beispiel 1

92 g (6R,S)-5-Methyl-tetrahydrofolsäure in 600 ml Wasser wurden mit 51 g N-Ethyl-2-aminomethylpyrrolidin versetzt. Das Reaktionsgemisch wurde auf 60°C erwärmt und 1 h bei dieser Temperatur stark gerührt. Nach dem Filtrieren wurde das Filtrat auf 20°C abgekühlt und das Kristallisat (36 g [α]_{D}²⁰: +24,5° (c=1 in 0,6 % Trilon B)) angesaugt.

Durch Suspendieren des so erhaltenen Salzes in 220 ml Wasser, Zugabe von 15 g Calciumchlorid und Erwärmen auf 35°C erhielt man daraus nach Abkühlen auf 17°C 30 g (6S)-Calcium-5-methyl-tetrahydrofolat x 5 H₂O, [α]_{D}²⁰: +34,5° (C = 1 in 0,6 % ®Trilon B). Die optische Reinheit des Produkts lag bei über 99 %.

### Beispiel 2

117,5 g (6R,S)-5-Methyl-tetrahydrofolsäure-Calciumsalz wurden unter Stickstoff in 100 ml Wasser suspendiert und zur Komplexbindung des Calciums mit 58,45 g Ethylendiamintetraessigsäure versetzt. Das Gemisch wurde nach Zugabe von 51,2 g (-)-N-Ethyl-2-aminomethylpyrrolidin auf 85°C erwärmt. Nach Abkühlen auf 60°C wurde das schwer lösliche 6R-Salz abgesaugt. Die Mutterlauge wurde auf 20°C abgekühlt und das so erhaltene Kristallisat abgesaugt und mit Wasser gewaschen. Man erhielt 46,2 g reines Pyrrolidinsalz der (6S)-5-Methyltetrahydrofolsäure.

### Beispiel 3

Das Beispiel 2 wurde wiederholt, jedoch wurde anstelle des (-)- das (+)-N-Ethyl-2-aminomethylpyrrolidin verwendet. Man erhielt 25 g Pyrrolidinsalz der (6S)-5-Methyl-tetrahydrofolsäure.

### Beispiel 4

Das Beispiel 2 wurde wiederholt, jedoch wurde anstelle des (-)- das (±)-N-Ethyl-aminomethylpyrrolidin verwendet. Man erhielt 28 g Pyrrolidinsalz der (6S)-5-Methyl-tetrahydrofolsäure.

## Patentansprüche

1. Verfahren zur Herstellung von (6S)-5-Methyl-tetrahydrofolsäure durch Racemattrennung der (6R,S)-5-Methyl-tetrahydrofolsäure mit Hilfe einer Base, dadurch gekennzeichnet, daß man als Base N-Ethyl-2-aminomethylpyrrolidin oder dessen optische Isomere verwendet.

## Claims

1. A process for preparing (65)-5-methyltetrahydrofolic acid by resolution of racemic (6R,S)-5-methyltetrahydrofolic acid using a base, wherein the base is N-ethyl-2-aminomethylpyrrolidine or its optical isomers.

## Revendications

1. Procédé de préparation d'acide (6S)-5-méthyl-tétrahydrofollique par séparation de racémates de l'acide (6R,S)-5-méthyl-tétrahydrofollique à l'aide d'une base, caractérisé par le fait qu'on utilise, comme base, la N-éthyl-2-aminométhylpyrrolidine ou ses isomères optiques.
